(19) Europäisches Patentamt
European Patent Office
Office européen des brevets

(11) EP 0 583 433 B1

(12) **EUROPÄISCHE PATENTSCHRIFT**

(45) Veröffentlichungstag und Bekanntmachung des Hinweises auf die Patenterteilung:
**12.05.1999 Patentblatt 1999/19**

(21) Anmeldenummer: **93902020.2**

(22) Anmeldetag: **01.02.1993**

(51) Int Cl.[6]: **C07F 9/50**, C07F 9/6568

(86) Internationale Anmeldenummer:
**PCT/CH93/00025**

(87) Internationale Veröffentlichungsnummer:
**WO 93/15090 (05.08.1993 Gazette 1993/19)**

(54) **DIPHOSPHINLIGANDEN**
DIPHOSPHINE LIGANDS
LIGANDS DIPHOSPHINIQUES

(84) Benannte Vertragsstaaten:
**AT BE CH DE DK ES FR GB IT LI NL**

(30) Priorität: **31.01.1992 CH 289/92**
**16.04.1992 CH 1270/92**
**18.05.1992 CH 1582/92**
**19.06.1992 CH 1944/92**

(43) Veröffentlichungstag der Anmeldung:
**23.02.1994 Patentblatt 1994/08**

(73) Patentinhaber: **F. HOFFMANN-LA ROCHE AG**
**4002 Basel (CH)**

(72) Erfinder:
- **FORICHER, Joseph**
  **F-68200 Mulhouse (FR)**
- **SCHMID, Rudolf**
  **CH-4144 Arlesheim (CH)**

(74) Vertreter: **Cottong, Norbert A.**
**F.Hoffmann-La Roche AG**
**Patent Department (PLP),**
**124 Grenzacherstrasse**
**4070 Basel (CH)**

(56) Entgegenhaltungen:
**EP-A- 0 104 375** **EP-A- 0 398 132**
**EP-A- 0 408 338**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft neue, racemische und optisch aktive Phosphorverbindungen der allgemeinen Formel

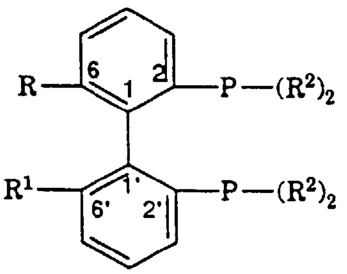

I

worin

R und $R^1$ jeweils unabhängig voneinander Hydroxy, oder eine geschützte Hydroxygruppe bedeuten, wobei die Schutzgruppen Etherbildende Gruppen wie Benzyl-, Allyl-, Benzyloxymethyl-, $C_{1-5}$-Alkoxymethyl- oder 2-Methoxyethoxymethyl-Gruppen sind;

$R^2$ $C_1$-$C_5$-Alkyl, $C_1$-$C_7$-Cydoalkyl; unsubstituiertes Phenyl, ortho-, meta- oder para-einfach oder mehrfach substituiertes Phenyl bedeutet, wobei die Substituenten Phenyl, $C_1$-$C_5$-Alkyl, $C_1$-$C_5$-Alkoxy, Di-$C_1$-$C_5$-Alkylamino, Fluor, Tri-$C_1$-$C_5$-Alkylsilyl oder Naphthyl bedeuten; oder beide Reste $R^2$ zusammen mit dem Phosphoratom eine Gruppe der Formel

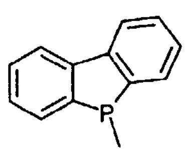

bedeuten;

mit der Massgabe, dass mindestens eine der Gruppen R oder $R^1$ Hydroxy bedeutet.

Die Erfindung betrifft ferner die Herstellung der Phosphorverbindungen der Formel I, sowie deren Verwendung für enantioselektive Reaktionen, wie z.B. asymmetrische Hydrierungen, enantioselektive Wasserstoffverschiebungen in prochiralen, allylischen Systemen, und dergleichen. Die Verbindungen der Formel I, worin R und $R^1$ beide Hydroxygruppen darstellen, können zudem als Ausgangsmaterial zur Herstellung weiterer Diphosphine verwendet werden, z.B. von Verbindungen der Formel I, worin R und/oder $R^1$ eine geschützte Hydroxygruppe darstellen.

In der europäischen Patentanmeldung EP-A 0 398 132 werden Diphenylphosphin- oder Di-p-tolylphosphin-Verbindungen beschrieben wie z.B. (6,6-Dimethoxybiphenyl-2,2'-diyl)bis(diphenylphosphin) oder (6,6-Dimethoxybiphenyl-2 ,2'-diyl)bis(di-p-tolylphosphin).

In der europäischen Patentanmeldung EP-A 0 104 375 werden Diphenylphosphin-Verbindungen beschrieben wie z.B. 6,6-Dimethylbiphenyl-2,2'-diyl)bis (diphenylphosphin).

In der europäischen Patentanmeldung EP-A 0 408 338 werden asymmetrische Synthesen von Carbonsäuren und Estern aus den jeweiligen ungesättigten Vorstufen mittels einer Diphosphinverbindung des Typs 2,2'-bis (Dicyclohexylphosphinyl)-6,6'-dimethyl-1,1'-biphenyl beschrieben.

In der internationalen Patentanmeldung WO-A-92 16535 werden Diphosphin-Verbindungen als Zwischenprodukte zur Herstellung von Phosphonsäurediphenylestern beschrieben.

Keine der oben genannten Patentanmeldungen beschreibt Phosphorverbindungen der Formel I, die mindestens eine freie Hydroxy-Gruppe tragen.

Der Ausdruck "niederes Alkyl" bedeutet, im Rahmen der vorliegenden Erfindung, geradkettige oder verzweigte Alkylgruppen mit 1 bis 5 Kohlenstoffatomen wie Methyl, Aethyl, Propyl, Isopropyl, Butyl, Isobutyl, tert. Butyl, Pentyl, Isopentyl und dergleichen. Der Ausdruck "niederes Alkoxy" bedeutet Gruppen, in denen der Alkylrest die vorhergehende Bedeutung hat. Als Schutzgruppen für die Hydroxygruppe kommen, im Rahmen der vorliegenden Erfindung, insbesondere in Frage die üblichen Aether-bildenden Gruppen, wie z.B. Benzyl, Allyl, Benzyloxymethyl, niederes Alkoxymethyl oder auch 2-Methoxyäthoxymethyl und dergleichen. Der Ausdruck "Cycloalkyl" bedeutet hier drei- bis siebengliedrige Ringe wie Cyclopropyl, Cyclobutyl, Cyclopentyl, Cyclohexyl und Cycloheptyl, insbesondere Cyclopentyl und Cyclohexyl. Der Ausdruck "Aryl" bedeutet im Rahmen der vorliegenden Erfindung insbesondere den Phenylrest, welcher sowohl unsubstituiert als auch in ortho, meta oder para-Stellung oder auch mehrfach substituiert sein kann. Als Substituenten kommen hier in Frage Phenyl, niedere Alkyl- oder niedere Alkoxygruppen, vorzugsweise Methyl- oder Methoxygruppen, oder auch Di-niederes Alkylamino, vorzugsweise Dimethylaminogruppen, sowie Fluor oder auch Trialkylsilyl, wie Trimethylsilyl und dergleichen. Der Ausdruck kann zudem auch Naphthyl bedeuten.

Die Phosphorverbindungen der Formel I können sowohl in racemischer als auch in optisch aktiver Form vorliegen. Bevorzugte Verbindungen der Formel I sind solche, worin R Hydroxy und $R^1$ eine geschützte Hydroxygruppe oder eine Alkoxygruppe, insbesondere eine Benzyloxy- oder eine Isopropyloxygruppe darstellen, sowie auch diejenigen worin R und $R^1$ gleich sind, insbesondere diejenigen worin R und $R^1$ beide eine Hydroxygruppe darstellen. Ferner sind auch Verbindungen bevorzugt, worin $R^2$ Phenyl, Isopropyl, Cyclopentyl oder Cyclohexyl darstellt. Besonders bevorzugte Verbindungen der Formel I sind:

(R)- oder (S)-6,6'-Dihydroxybiphenyl-2,2'-diyl)bis (diphenylphosphin),

(R)- oder (S)-6-Hydroxy-6'-isopropoxybiphenyl-2,2'-diyl)-bis(diphenylphosphin).

Die erfindungsgemässen Verbindungen der Formel I können in an sich bekannter Weise hergestellt werden. Dies kann z.B. dadurch erfolgen, dass man eine racemische oder optisch aktive Verbindung der allgemeinen Formel

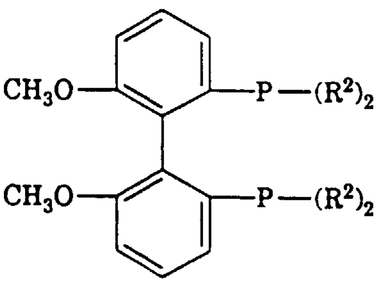

II

worin $R^2$ die obige Bedeutung hat,

einer Aetherspaltung unterwirft. Hierbei erhält man Verbindungen der Formel I, worin R und $R^1$ Hydroxy bedeuten. Derartige Verbindungen wiederum können leicht in an sich bekannter Weise in Verbindungen der Formel I übergeführt werden, worin R oder $R^1$ eine geschützte Hydroxygruppe darstellen oder auch in solche Verbindungen, worin R Hydroxy bedeutet und $R^1$ niederes Alkoxy darstellt.

Die Aetherspaltung einer Verbindung der Formel I, worin R niederes Alkoxy darstellt, karn in an sich bekannter Weise durchgeführt werden, z.B. mittels Bortribromid und dergleichen. Dies erfolgt zweckmässig in einem inerten organischen Lösungsmittel, z.B. in einem halogenierten Kohlenwasserstoff wie etwa Methylenchlorid und dergleichen, und bei einer Temperatur von etwa 30° bis etwa -78°C.

Die Einführung einer Hydroxyschutzgruppe in eine Verbindung der Formel I, worin R und $R^1$ Hydroxy darstellen, kann in an sich bekannter Weise erfolgen, z. B. durch Umsetzung mit einem Schutzgruppen-Halogenid, z.B. Benzylchlorid und dergleichen. Diese Umsetzung erfolgt zweckmässig in einem inerten organischen Lösungsmittel in Gegenwart einer anorganischen Base wie z.B. $K_2CO_3$, NaH oder KOH. Als Lösungsmittel kommen in Frage z.B. Aceton, N,N-Dimethylformamid, oder im Falle von KOH insbesondere Dimethylsulfoxid. Die Umsetzung erfolgt auch zweckmässig bei einer Temperatur von etwa -10°C bis etwa 60°C, bevorzugt bei etwa 0°C bis etwa 25°C.

Die Ueberführung einer Verbindung der Formel I, worin R und $R^1$ Hydroxy bedeuten in eine Verbindung der Formel I, worin $R^1$ niederes Alkoxy darstellt kann ebenfalls in an sich bekannter Weise erfolgen, z. B. durch Monoalkylierung. Dies erfolgt zweckmässig mit einem niederen Alkylhalogenid in einem inerten organischen Lösungsmittel in Gegenwart einer anorganischen Base wie z.B. $K_2CO_3$, NaH oder KOH. Als Lösungsmittel kommen in Frage z.B. Aceton, N,N-Dimethylformamid, oder im Falle von KOH insbesondere Dimethylsulfoxid. Die Umsetzung erfolgt auch zweckmässig bei einer Temperatur von etwa -10°C bis etwa 60°C, bevorzugt bei etwa 0°C bis etwa 25°C.

Die als Ausgangsmaterial verwendeten Verbindungen der Formel II sind bekannte Verbindungen oder Analoge bekannter Verbindungen, welche leicht in zur Herstellung bekannten Verbindungen analoger Weise hergestellt werden können, beispielsweise gemäss Helv. Chim. Acta 74, 370 (1991).

Sämtliche vorhergehend erwähnten Reaktionen werden zweckmässig unter Inertgas, wie z.B. Argon oder Stickstoff, durchgeführt.

Die erfindungsgemässen Phosphorverbindungen der Formel I bilden Komplexe mit Uebergangsmetallen wie etwa Metallen der Gruppe VIII, insbesondere mit Ruthenium, Rhodium und Iridium, welche als Katalysatoren bei asymmetrischen Hydrierungen und auch für enantioselektive Wasserstoffverschiebungen in prochiralen, allylischen Systemen verwendbar sind. Für die erwähnten Hydrierungen sind Ruthenium- und Rhodium-Komplexe bevorzugt, während für Isomerisierungen Rhodium-Komplexe bevorzugt sind. Diese Katalysatoren, d.h. die Komplexe aus einem Metall der Gruppe VIII und den Phosphorverbindungen der Formel I sind neu und ebenfalls Gegenstand der vorliegenden Erfindung.

Die in Frage stehenden Komplexe können in an sich bekannter Weise hergestellt werden, z.B. indem man eine Verbindung der Formel I mit einer Verbindung, welche ein Metall der Gruppe VIII abgeben kann, in einem geeigneten, inerten organischen oder wässrigen Lösungsmittel umsetzt. Als geeignete, z.B. Rhodium abgebende Verbindungen können beispielsweise genannt werden, organische Rhodium-Komplexe mit Aethylen, Propylen und dergleichen, sowie mit bis-Olefinen, z.B. (Z,Z)-1,5-Cyclooctadien, 1,5-Hexadien, Bicyclo[2.2.1]hepta-2,5-dien oder mit weiteren Dienen, welche mit Rhodium leicht lösliche Komplexe bilden. Bevorzugte, Rhodium abgebende Verbindungen sind z. B. Di-μ-chloro-bis[$\eta^4$-(Z,Z)-1,5-cyclooctadien]dirhodium(I), Di-μ-chloro-bis[$\eta^4$-norbornadien]dirhodium(I), Di-μ-Trifluoracetato-bis[$\eta^4$-(Z,Z)-1,5-cyclooctadien]dirhodium(I), Bis[$\eta^4$-(Z,Z)-1,5-cyclooctadien]rhodiumtetrafluorborat oder Bis[$\eta^4$-(Z,Z)-cyclooctadien]rhodiumperchlorat. Als Iridium abgebende Verbindung kann beispielsweise Di-μ-chloro-bis[$\eta^4$-(Z,Z)-1,5-cyclooctadien]diiridium(I) genannt werden.

Die in Frage stehenden Ruthenium-Komplexe können z.B. durch folgende Formel dargestellt werden

$$Ru(Z)_2L \qquad III$$

worin Z Halogen oder die Gruppe A-COO, A niederes Alkyl, Aryl, halogeniertes niederes Alkyl oder halogeniertes Aryl und L einen chiralen Diphosphinliganden der Formel I darstellen.

Diese Komplexe können im Prinzip in an sich bekannter Weise hergestellt werden. Zweckmässig und bevorzugt werden Ruthenium-Komplexe beispielsweise dadurch hergestellt, dass man einen Komplex der Formel

$$[Ru(Z^1)_2L^1{}_m]_p \cdot (H_2O)_q \qquad IV$$

worin $Z^1$ Halogen oder eine Gruppe $A^1$-COO, $A^1$ niederes Alkyl oder halogeniertes niederes Alkyl, $L^1$ einen neutralen Liganden, m die Zahl 1, 2 oder 3, p die Zahl 1 oder 2 und q die Zahl 0 oder 1 darstellen,
mit einem chiralen Diphosphinliganden der Formel I umsetzt, oder, dass man einen Ruthenium-Komplex der Formel

$$Ru(CF_3COO)_2L \qquad V$$

worin L einen chiralen Diphosphinliganden der Formel I darstellt,
mit einem das Anion Z abgebenden Salz, worin Z obige Bedeutung hat, umsetzt.

Der Ausdruck "neutraler Ligand" bedeutet, im Rahmen der vorliegenden Erfindung, einen leicht austauschbaren Liganden wie etwa ein Diolefin, z.B. Norbornadien, (Z,Z)-1,5-Cyclooctadien usw., oder auch ein Nitril wie Acetonitril, Benzonitril und dergleichen. Falls m die Zahl 2 oder 3 darstellt, können die Liganden gleich oder auch verschieden sein.

Die Rutheniumkomplexe der Formel IV sind bekannte Substanzen oder Analoge bekannter Substanzen, welche leicht in zur Herstellung der bekannten analoger Weise erhalten werden können, beispielsweise gemäss Albers, M.O. et al., J. Organomet. Chem. 272, C62-C66 (1984).

Die Umsetzung eines Rutheniumkomplexes der Formel IV mit einem chiralen Diphosphinliganden der Formel I kann in an sich bekannter Weise durchgeführt werden. Diese Reaktion kann zweckmässig in einem inerten organischen Lösungsmittel erfolgen. Als Beispiele derartiger Lösungsmittel können genannt werden, z.B. Aether wie Tetrahydrofuran oder Dioxan, Ketone wie etwa Aceton, niedere Alkohole wie etwa Methanol, Aethanol usw., halogenierte Kohlenwasserstoffe wie Methylenchlorid, Chloroform und dergleichen, oder auch Gemische derartiger Lösungsmittel. Die Reaktion kann zudem bei einer Temperatur zwischen etwa 0°C und etwa 100°C, und vorzugsweise zwischen etwa 15°C und etwa 60°C erfolgen, jedoch unter striktem Ausschluss von Sauerstoff.

Die Umsetzung eines Rutheniumkomplexes der Formel V (erhältlich aus einem Komplex der Formel IV) mit einem das Anion Z enthaltenden Salz kann in an sich bekannter Weise erfolgen. Der Ausdruck "ein das Anion Z abgebendes Salz" bedeutet im Rahmen der vorliegenden Erfindung beispielsweise Ammoniumsalze, Alkalimetallsalze oder andere geeignete Metallsalze. Um die Löslichkeit derartiger Salze zu verbessern, können in gewissen Fällen auch Kronenäther oder dergleichen zugesetzt werden.

Wie bereits eingangs erwähnt, sind die erfindungsgemässen Phosphorverbindungen, in Form von Komplexen mit Metallen der Gruppe VIII, und insbesondere Rhodium und Ruthenium, u.a. verwendbar für asymmetrische Hydrierungen. Als besonders geeignete Substrate können in diesem Zusammenhang insbesondere genannt werden, Allylalkohole wie z.B. Geraniol, 6,7-Dihydrogeraniol, 6,7-Dihydrofarnesol, 6,7,10,11-Tetrahydrofarnesol, (E)-3-(p-tert.Butylphenyl)-2-methyl-2-propen-2-ol und dergleichen, Enamide wie z.B. (Z)-2-Acetyl-1-(p-methoxybenzyliden)-1,2,3,4,5,6,7,8-octahydroisochinolin sowie auch funktionalisierte Ketone wie β-Ketoester, z.B. Acetessigsäuremethyl- oder äthylester usw. oder auch 2-Pyridylketone wie z.B. 2-Acetylpyridin, 2-Pyridyl-2,8-bis(trifluormethyl)-4-chinolylketon und dergleichen.

Bei der Durchführung derartiger Hydrierungen, können diese Komplexe zuerst hergestellt und dann zu einer Lösung der zu hydrierenden Substanz gegeben werden. Alternativ können sie jedoch auch in situ hergestellt werden, z.B. auch in Gegenwart einer zu hydrierenden Substanz.

Die asymmetrische Hydrierung kann in einem geeigneten, unter den Reaktionsbedingungen inerten organischen Lösungsmittel erfolgen. Als derartige Lösungsmittel können insbesondere genannt werden, aromatische Kohlenwasserstoffe wie Benzol, Toluol usw., niedere Alkohole wie z.B. Methanol oder Aethanol, Ester wie Aethylacetat, halogenierte Kohlenwasserstoffe wie Methylenchlorid, Chloroform und dergleichen, cyclische Aether wie Tetrahydrofuran oder Dioxan, und dergleichen, oder Gemische derartiger Lösungsmittel. Auch Wasser oder wässrige Gemische mit organischen Lösungsmitteln können verwendet werden.

Das Verhältnis von Metall zu Ligand L liegt zweckmässig zwischen etwa 0,05 und etwa 5 Mol, bzw. zwischen etwa 0,5 und etwa 2 Mol, vorzugsweise bei etwa 1 Mol Metall pro Mol Ligand. Das Verhältnis von Metall, in den Komplexen wie z.B. der Formel III, zu den zu hydrierenden Substanzen liegt zweckmässig zwischen etwa 0,0005 und etwa 1 Mol %, vorzugsweise zwischen etwa 0,002 und etwa 0,1 Mol %.

Die asymmetrische Hydrierung mit Komplexen wie z.B. der Formel III erfolgt zweckmässig bei einer Temperatur von etwa 0°C bis etwa 150°C, in Abhängigkeit des verwendeten Substrates. Diese Hydrierung erfolgt zweckmässig auch unter Druck, vorzugsweise bei einem Druck von etwa 2 bis etwa 200 bar, besonders bevorzugt von etwa 10 bis etwa 100 bar.

Die Phosphorverbindung der Formel I, worin R und/oder $R^1$ Hydroxy darstellt, sind neben ihrer wie vorgehend erwähnten Verwendung zudem von Interesse indem sie, sowie auch entsprechende Komplexe mit Metallen der Gruppe VIII, aus Reaktionsgemischen leicht durch Adsorption an geeigneten Ionentauschern entfernt werden können.

Die folgenden Beispiele dienen zur Illustrierung der Erfindung und stellen in keiner Weise irgendeine Beschränkung dar. In diesen Beispielen haben die gewählten Abkürzungen folgende Bedeutung:

DC Dünnschichtchromatographie

GC Kapillar-Gaschromatographie

e.e. Enantiomeric Excess.

RT Raumtemperatur

Alle Temperaturen sind in °Celsius angegeben.

Beispiel 1

Herstellung von (R)- bzw. (S)-(6,6'-Dihydroxybiphenyl-2,2'-diyl)-bis(diphenylphosphin [(R)- bzw. (S)-HOBIPHEP]

Eine Lösung von 11,64 g (19,98 mMol) (R)-(6,6'-Dimethoxybiphenyl-2,2'-diyl)bis(diphenylphosphin)[= (R)-MeOBIPHEP) in 200 ml $CH_2Cl_2$ (über Molekularsieb getrocknet) wurde unter Argon auf -78° abgekühlt. Eine Lösung von 50 ml (50 mMol) 1M $BBr_3$ in $CH_2Cl_2$ wurde dann langsam zugefügt, wobei gegen Ende der Zugabe ein weisser Niederschlag entstand. Das Gemisch wurde sodann auf Raumtemperatur aufgewärmt und während 24 Stunden gerührt. Nach Hydrolyse mit 400 ml gesättigtem $NH_4Cl$ während 1 Stunde, Phasentrennung, Trocknen der organischen Phase ($CaCl_2$) und Eindampfen erhielt man 12,5 g eines gelben Pulvers. Nach Chromatographie an Silicagel (90 g, Hexan/$CH_2Cl_2$ 1:1, Hexan/Aethylacetat 9:1 $\rightarrow$ 3:2) erhielt man 8,26 g (R)-HOBIPHEP als weisses Pulver. Smp. 198,2°-199,4°. $[\alpha]_D^{20}$ = -34,7° (c = 0,9, $CHCl_3$).

Die Umsetzung von 0,65 g (1,1 mMol) (S)-MeOBIPHEP mit 2,2 ml (2,2 mMol) 1M $BBr_3$ in $CH_2Cl_2$ in zum Vorherigen analoger Weise ergab 0,35 g (S)-HOBIPHEP als weisses Pulver. Smp. 199,5°-200,5°. $[\alpha]_D^{20}$ = +33,9° (c = 1, $CHCl_3$).

Beispiel 3

Eine Lösung von 2,0 g (3,61 mMol) (R)-(6,6'-Dihydroxybiphenyl-2,2'-diyl)bis(diphenylphosphin) [hergestellt gemäss Beispiel 1] in 40 ml Dimethylsulfoxid wurde, unter Argon, während 30 Minuten mit 0,572 g (10,19 mMol) pulverisierter 80%iger KOH bei RT gerührt. Dann wurden 1,13 ml (12,03 mMol) Isopropylbromid tropfenweise zugesetzt und das Gemisch weitere 23 Stunden bei RT gerührt. Durch Hydrolyse mit 30 ml gesättigter $NH_4Cl$-Lösung, Extraktion mit ca. 175 ml Aether, Trocknen der organischen Phase ($Na_2SO_4$) und Eindampfen erhielt man 2,41 g eines weissen Pulvers. Nach Chromatographie an 70 g Silicagel (Hexan/$CHCl_2$) erhielt man 1,18 g (R)-(6,6'-Diisopropyloxybiphenyl-2,2'-diyl)bis(diphenylphosphin) als weissen Feststoff. Weitere Elution ergab dann 0,45 g (R)-(6-Hydroxy-6'-isopropyloxybiphenyl-2,2'-diyl)bis(diphenylphosphin) [(R)-IpropO/HOBIPHEP] als weissen Feststoff. Smp. 76,1-77°; $[\alpha]_D^{20}$ = -46,0° (c=1, $CHCl_3$).

Beispiel 4

a) Eine Suspension von 0,375 g (0,43 mMol) $[Ru(CF_3COO)_2(COD)]_2$ in 16 ml Diäthyläther/Tetrahydrofuran (1:1) wurde mit 0,466 g (0,84 mMol) (R)-HOBIPHEP (hergestellt gemäss Beispiel 1) über Nacht bei 40° gerührt. Nach Entfernen des Lösungsmittels wurde der Rückstand im Vakuum getrocknet, und der feste Rückstand unter Rühren zweimal mit je 10 ml Pentan gewaschen. Nach Trocknen im Vakuum erhielt man 0,72 g $[Ru(CF_3COO)_2$((R)-HOBIPHEP)].

Eine Suspension von 3,0 g (3,40 mMol) [Ru $(CF_3COO)_2$((R)-HOBIPHEP)] in 30 ml Methanol wurde mit 1,0 g (12,0 mMol) Natriumacetat versetzt und 2 Stunden bei 45° gerührt. Nach Entfernen des Lösungsmittels wurde der Rückstand im Vakuum getrocknet und anschliessend auf eine Fritte gegeben. Das Produkt wurde mit 60 ml Methylenchlorid extrahiert, dann das Lösungsmittel entfernt und der Rückstand mit 30 ml Pentan verrührt. Die überstehende Lösung wurde abpipettiert und das verbleibende Pulver im Vakuum getrocknet. Man erhielt 2,5 g $Ru(CH_3COO)_2$[(R)-HOBIPHEP].

b) In einer Handschuhbox wurde ein 500 ml-Autoklav mit 15,0 g (50,4 mMol) (Z)-2-Acetyl-1-(p-methoxybenzyliden)-1,2,3,4,5,6,7,8-octahydroisochinolin, 170 ml Methanol und 3,9 mg (0,0050 mMol) Ru $(CH_3COO)_2$[(R)-HOBIPHEP] als Katalysator beladen. Die Hydrierung wurde bei 100° und 35 bar während 22 Stunden durchgeführt. Der Umsatz betrug 98,5%. Ein 2 g Produkt enthaltender Teil der Hydrierlösung wurde eingedampft und der Rückstand in Diäthyläther gelöst. Zur Abtrennung der Katalysators wurde die Aetherlösung durch ein Kieselgel-Polster filtriert. Eindampfen des Filtrates ergab 1,97 g (S)-2-Acetyl-1-(p-methoxybenzyl)-1,2,3,4,5,6,7,8-octahydroisochinolin als gelbliche Kristalle von 95,9% e.e.

Zur Bestimmung des e.e.-Wertes wurde das Produkt in einem Gemisch von Aethylenglykol und 40% wässriger Kaliumhydroxid-Lösung bei 170° während 18 Stunden hydrolysiert. Das gebildete Amin wurde mit (-)-Camphansäurechlorid in Pyridin/4-Dimethylaminopyridin zum Gemisch der diastereomeren Amide umgesetzt, und letzteres mittels GC analysiert.

<u>Beispiel 5</u>

In einer Glove-Box (02-Gehalt <1 ppm) wurde in einem 50 ml Messkolben eine Katalysatorlösung, durch Lösen von 9,7 mg (0,02 mMol) Dichloro-bis-(1,5-cyclooctadien)dirhodium und 21,9 mg (0,04 mMol) (R)-HOBIPHEP in 50 ml Toluol hergestellt. 5 ml dieser Katalysatorlösung wurden zu einer Lösung von 16,1 g (78,85 mMol) (E)-3-(p-tert.Butylphenyl)-2-methyl-2-propen-1-ol [(E)-Dehydroliliol] in 145 ml Toluol in einen 500 ml Autoklav gegeben. Die Hydrierung wurde bei 100°, einem konstanten Druck von 60 bar und unter intensivem Rühren durchgeführt. Nach 21 Stunden betrug der Umsatz 99,9%. Die hellgelbe Hydrierlösung wurde aus dem Autoklaven gespült und am Rotationsverdampfer bei 60°/17 mbar eingedampft. Der Rückstand wurde bei 140°/0,01 mbar destilliert. Man erhielt 16,1 g (99,0%) (S)-3-(p-tert.Butylphenyl)-2-methyl-1-propanol [(S)-Liliol], als farbloses Oel, mit einer enantiomeren Reinheit von 96,2% e.e.

Zur Bestimmung des e.e.-Wertes wurde das Produkt in Methylenchlorid mit (R)- oder (S)-6-Methoxy-2,5,7,8-tetramethylchroman-2-carbonsäure, Dicyclohexylcarbodiimid und 4-Dimethylaminopyridin in die diastereomeren Ester überführt und gaschromatographisch analysiert.

<u>Beispiel 6</u>

In zu Beispiel 5 analoger Weise wurde die Katalysatorlösung mit (R)-IpropO/HOBIPHEP als Ligand hergestellt und die Hydrierung unter denselben Bedingungen durchgeführt. Nach 21 Stunden betrug der Umsatz 99,9%. Die Hydrierlösung wurde wie in Beispiel 5 aufgearbeitet. Man erhielt 16,1 g (99,0%) (S)-Liliol, als farbloses Oel, mit einer enantiomeren Reinheit von 95,1% e.e.

## Patentansprüche

1. Racemische und optisch aktive Phosphorverbindungen der allgemeinen Formel

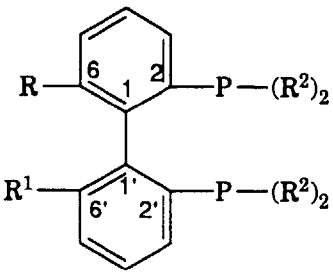

I

worin

R und $R^1$ jeweils unabhängig voneinander Hydroxy, oder eine geschützte Hydroxygruppe bedeuten, wobei die Schutzgruppen Ether-bildende Gruppen wie Benzyl-, Allyl-, Benzyloxymethyl-, $C_{1-5}$-Alkoxymethyl- oder 2-Methoxyethoxymethyl-Gruppen sind;

$R^2$ $C_1$-$C_5$-Alkyl, $C_1$-$C_7$-Cycloalkyl; unsubstituiertes Phenyl, ortho-, meta- oder para-einfach oder mehrfach substituiertes Phenyl bedeutet, wobei die Substituenten Phenyl, $C_1$-$C_5$-Alkyl, $C_1$-$C_5$-Alkoxy, Di-$C_1$-$C_5$-Alkylamino, Fluor, Tri-$C_1$-$C_5$-Alkylsilyl oder Naphthyl bedeuten; oder beide Reste $R^2$ zusammen mit dem Phosphoratom eine Gruppe der Formel

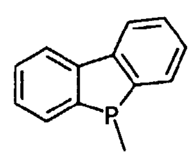

bedeuten;
mit der Massgabe, dass mindestens eine der Gruppen R oder $R^1$ Hydroxy bedeutet.

2. Racemische und optisch aktive Phosphorverbindungen der Formel I gemäss Anspruch 1, worin R und $R^1$ beide Hydroxy bedeuten.

3. Racemische und optisch aktive Phosphorverbindungen der Formel I gemäss Anspruch 1, worin R Hydroxy und $R^1$ eine geschützte Hydroxygruppe darstellen.

4. Racemische und optisch aktive Phosphorverbindungen der Formel I gemäss Anspruch 1 oder 3, worin R Hydroxy und $R^1$ eine Benzyloxy- oder eine Isopropyloxygruppe darstellen.

**5.** R)- oder (S)-6,6'-Dihydroxybiphenyl-2,2'-diyl)bis (diphenylphosphin).

**6.** R)- oder (S)-6-Hydroxy-6'-isopropyloxybiphenyl-2,2'-diyl)-bis(diphenylphosphin).

**7.** Komplexe von Phosphorverbindungen der Formel I gemäss Anspruch 1 mit einem Metall der Gruppe VIII.

**8.** Komplexe gemäss Anspruch 8, worin als Metall der Gruppe VIII, Ruthenium, Rhodium oder Iridium enthalten sind.

**9.** Komplexe gemäss Anspruch 8, worin als Metall der Gruppe VIII, Ruthenium oder Rhodium enthalten sind.

**10.** Verwendung der Phosphorverbindungen der Formel I gemäss Anspruch 1, in Form ihrer Komplexe mit einem Metall der Gruppe VIII als Katalysator bei asymmetrischen Hydrierungen und für enantioselektive Wasserstoffverschiebungen in prochiralen, allylischen Systemen.

## Claims

**1.** Racemic and optically active phosphorus compound of the general formula

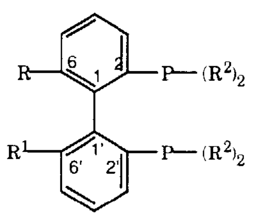

I

wherein

R and $R^1$ each independently signify hydroxy or a protected hydroxy group, with the protecting groups being ether-forming groups such as benzyl, allyl, benzyloxymethyl, $C_{1-5}$-alkoxymethyl or 2-methoxyethoxymethyl groups;

$R^2$ signifies $C_1$-$C_5$-alkyl, $C_1$-$C_7$-cycloalkyl; unsubstituted phenyl, ortho-, meta- or para- mono- or multiply-substituted phenyl, with the substitutents being phenyl, $C_1$-$C_5$-alkyl, $C_1$-$C_5$-alkoxy, di-$C_1$-$C_5$-alkylamino, fluorine, tri-$C_1$-$C_5$-alkylsilyl or naphthyl; or both residues $R^2$ together with the phosphorus atom signify a group of the formula

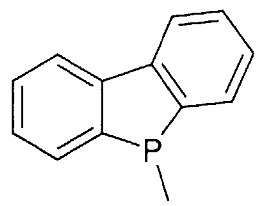

;

with the proviso that at least one of the groups R or $R^1$ signifies hydroxy.

**2.** Racemic and optically active phosphorus compounds of formula I according to claim 1, wherein R and $R^1$ both signify hydroxy.

**3.** Racemic and optically active phosphorus compounds of formula I according to claim 1, wherein R represents hydroxy and $R^1$ represents a protected hydroxy group.

**4.** Racemic and optically active phosphorus compounds of formula I according to claim 1 or 3, wherein R represents hydroxy and $R^1$ represents a benzyloxy group or an isopropyloxy group.

**5.** (R)- or (S)-(6,6'-Dihydroxybiphenyl-2,2'-diyl)bis (diphenylphosphine).

**6.** (R)- or (S)-(6-Hydroxy-6'-isopropyloxybiphenyl-2,2'-diyl)bis(diphenylphosphine).

**7.** Complexes of phosphorus compounds of formula I according to claim 1 with a metal of Group VIII.

**8.** Complexes according to claim 8, wherein ruthenium, rhodium or iridium is present as the metal of Group VIII.

**9.** Complexes according to claim 8, wherein ruthenium or rhodium is present as the metal of Group VIII.

**10.** The use of phosphorus compounds of formula I according to claim 1 in the form of their complexes with a metal of Group VIII as catalysts in asymmetric hydrogenations and for enantioselective hydrogen displacements in prochiral, allylic systems.

## Revendications

**1.** Composés phosphorés optiquement actifs et racémiques de formule générale

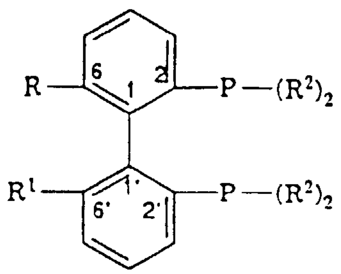

I

dans laquelle

R et $R^1$ représentent chacun, indépendamment l'un de l'autre, le groupe hydroxyle ou un groupe hydroxyle protégé, les groupes protecteurs étant des groupes éthérifiants tels que les groupes benzyle, allyle, benzyloxyméthyle, alcoxy($C_{1-5}$) méthyle ou 2-méthoxyéthoxyméthyle;

$R^2$ représente un groupe alkyle en $C_1$-$C_5$, cycloalkyle en $C_1$-$C_7$; phényle non substitué, phényle une ou plusieurs fois substitué en position ortho, méta ou para, les substituants étant des groupes phényle, alkyle en $C_1$-$C_5$, alcoxy en $C_1$-$C_5$, dialkyl($C_1$-$C_5$)amino, l'atome de fluor, des groupes trialkyl($C_1$-$C_5$)silyle ou naphtyle; ou les deux radicaux $R^2$ représentent conjointement avec l'atome de phosphore un groupe de formule

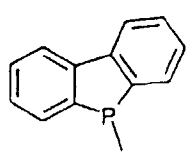

étant entendu qu'au moins un des groupes R et $R^1$ représente le groupe hydroxyle.

**2.** Composés phosphorés optiquement actifs et racémiques de formule I selon la revendication 1, dans lesquels R et $R^1$ représentent chacun le groupe hydroxyle.

**3.** Composés phosphorés optiquement actifs et racémiques de formule I selon la revendication 1, dans lesquels R représente le groupe hydroxyle et $R^1$ représente un groupe hydroxyle protégé.

**4.** Composés phosphorés optiquement actifs et racémiques de formule I selon la revendication 1 ou 3, dans lesquels R représente le groupe hydroxyle et $R^1$ représente le groupe benzyloxy ou isopropyloxy.

**5.** (R)- ou (S)-(6,6'-dihydroxybiphényl-2,2'-diyl)-bis (diphénylphosphine).

**6.** (R) - ou (S)-(6-hydroxy-6'-isopropyloxybiphényl-2,2'-diyl)-bis(diphénylphosphine).

**7.** Complexes de composés phosphorés de formule I selon la revendication 1, avec un métal du groupe VIII.

**8.** Complexes selon la revendication 8, dans lesquels le ruthénium, le rhodium ou l'iridium est contenu en tant que métal du groupe VIII.

**9.** Complexes selon la revendication 8, dans lesquels le ruthénium ou le rhodium est contenu en tant que métal du groupe VIII.

**10.** Utilisation des composés phosphorés de formule I selon la revendication 1, sous forme de leurs complexes avec un métal du groupe VIII, en tant que catalyseur dans des hydrogénations asymétriques et pour des migrations énantiosélectives d'hydrogène dans des systèmes allyliques prochiraux.